# EUROPEAN PATENT SPECIFICATION

(11) **EP 3 040 419 B1**
(45) Date of publication and mention of the grant of the patent: **14.08.2019**
(21) Application number: 14839952.0
(22) Date of filing: 27.08.2014
(51) Int. Cl.: C12N 15/12, C12P 21/02, C12N 15/85, C07K 14/745, C12N 9/06, C12N 9/64

(54) **METHOD FOR MASS PRODUCING HUMAN BLOOD COAGULATION FACTOR VII DERIVATIVE**
VERFAHREN ZUR MASSENERZEUGUNG EINES DERIVATS DES MENSCHLICHEN BLUTGERINNUNGSFAKTORS VII
PROCÉDÉ POUR LA PRODUCTION EN MASSE DE DÉRIVÉ DU FACTEUR DE COAGULATION SANGUINE VII HUMAIN

(30) Priority: 30.08.2013 KR 20130104308
(43) Date of publication of application: 06.07.2016
(73) Proprietor: Hanmi Pharm. Co., Ltd., Hwaseong-si, Gyeonggi-do 445-958 (KR)
(72) Inventor: KANG, Hee Chul, Hwaseong-si Gyeonggi-do 445-856 (KR); KIM, Jin Young, Chuncheon-si Gangwon-do 200-935 (KR); LEE, Byung Sun, Seoul 150-957 (KR); KIM, Hyun Uk, Busan 600-810 (KR); CHOI, In Young, Yongin-si Gyeonggi-do 448-755 (KR); KWON, Se Chang, Seoul 135-506 (KR)
(74) Representative: Mewburn Ellis LLP
(86) International application number: PCT/KR2014/007961
(87) International publication number: WO 2015/030479

(56) References cited:
- WO-A1-2008/048037
- WO-A2-2012/057527
- WO-A2-2013/051900
- CN-A- 102 321 668
- KR-A- 20080 034 364
- KR-A- 20110 090 604
- KR-A- 20120 043 657
- KR-A- 20130 037 659

<110> HANMI PHARM. CO., LTD.
<120> Method for mass production of Factor VII analog
<130> OPA14136-PCT
<150> KR10-2013-0104308
   <151> 2013-08-30
<160> 9
<170> KopatentIn 2.0
<210> 1
   <211> 34
   <212> DNA
   <213> Artificial Sequence
<220>
   <223> VIIBHISS F
<400> 1
   cccggatcca tggtctccca ggccctcagg ctcc 34
<210> 2
   <211> 31
   <212> DNA
   <213> Artificial Sequence
<220>
   <223> VIIXhoIAS R
<400> 2
   gggctcgagc tagggaaatg gggctcgcag g 31
<210> 3
   <211> 1335
   <212> DNA
   <213> Artificial Sequence
<220>
   <223> FactorVII DNA
<400> 3
   atggtctccc aggccctcag gctcctctgc cttctgcttg ggcttcaggg ctgcctggct     60
   gcagtcttcg taacccagga ggaagcccac ggcgtcctgc accggcgccg gcgcgccaac    120
   gcgttcctgg aggagctgcg gccgggctcc ctggagaggg agtgcaagga ggagcagtgc    180
   tccttcgagg aggcccggga gatcttcaag gacgcggaga ggacgaagct gttctggatt    240
   tcttacagtg atggggacca gtgtgcctca agtccatgcc agaatggggg ctcctgcaag    300
   gaccagctcc agtcctatat ctgcttctgc ctccctgcct tcgagggccg gaactgtgag    360
   acgcacaagg atgaccagct gatctgtgtg aacgagaacg gcggctgtga gcagtactgc    420
   agtgaccaca cgggcaccaa gcgctcctgt cggtgccacg aggggtactc tctgctggca        480
   gacggggtgt cctgcacacc cacagttgaa tatccatgtg gaaaaatacc tattctagaa        540
   aaaagaaatg ccagcaaacc ccaaggccga attgtggggg gcaaggtgtg ccccaaaggg        600
   gagtgtccat ggcaggtcct gttgttggtg aatggagctc agttgtgtgg ggggaccctg        660
   atcaacacca tctgggtggt ctccgcggcc cactgtttcg acaaaatcaa gaactggagg        720
   aacctgatcg cggtgctggg cgagcacgac ctcagcgagc acgacgggga tgagcagagc        780
   cggcgggtgg cgcaggtcat catccccagc acgtacgtcc cgggcaccac caaccacgac        840
   atcgcgctgc tccgcctgca ccagcccgtg gtcctcactg accatgtggt gcccctctgc        900
   ctgcccgaac ggacgttctc tgagaggacg ctggccttcg tgcgcttctc attggtcagc        960
   ggctggggcc agctgctgga ccgtggcgcc acggccctgg agctcatggt cctcaacgtg       1020
   ccccggctga tgacccagga ctgcctgcag cagtcacgga aggtgggaga ctccccaaat       1080
   atcacggagt acatgttctg tgccggctac tcggatggca gcaaggactc ctgcaagggg       1140
   gacagtggag gcccacatgc cacccactac cggggcacgt ggtacctgac gggcatcgtc       1200
   agctggggcc agggctgcgc aaccgtgggc cactttgggg tgtacaccag ggtctcccag       1260
   tacatcgagt ggctgcaaaa gctcatgcgc tcagagccac gcccaggagt cctcctgcga       1320
   gccccatttc cctag                                                        1335
<210> 4
   <211> 444
   <212> PRT
   <213> Artificial Sequence
<220>
   <223> FactorVII
<400> 4
   Met Val Ser Gln Ala Leu Arg Leu Leu Cys Leu Leu Leu Gly Leu Gln
    1               5                  10                  15
   Gly Cys Leu Ala Ala Val Phe Val Thr Gln Glu Glu Ala His Gly Val
               20                  25                  30
   Leu His Arg Arg Arg Arg Ala Asn Ala Phe Leu Glu Glu Leu Arg Pro
           35                  40                  45
   Gly Ser Leu Glu Arg Glu Cys Lys Glu Glu Gln Cys Ser Phe Glu Glu
       50                  55                  60
   Ala Arg Glu Ile Phe Lys Asp Ala Glu Arg Thr Lys Leu Phe Trp Ile
   65                  70                  75                  80
   Ser Tyr Ser Asp Gly Asp Gln Cys Ala Ser Ser Pro Cys Gln Asn Gly
                   85                  90                  95
   Gly Ser Cys Lys Asp Gln Leu Gln Ser Tyr Ile Cys Phe Cys Leu Pro
               100                 105                 110
   Ala Phe Glu Gly Arg Asn Cys Glu Thr His Lys Asp Asp Gln Leu Ile
           115                 120                 125
   Cys Val Asn Glu Asn Gly Gly Cys Glu Gln Tyr Cys Ser Asp His Thr
       130                 135                 140
   Gly Thr Lys Arg Ser Cys Arg Cys His Glu Gly Tyr Ser Leu Leu Ala
   145                 150                 155                 160
   Asp Gly Val Ser Cys Thr Pro Thr Val Glu Tyr Pro Cys Gly Lys Ile
                   165                 170                 175
   Pro Ile Leu Glu Lys Arg Asn Ala Ser Lys Pro Gln Gly Arg Ile Val
               180                 185                 190
   Gly Gly Lys Val Cys Pro Lys Gly Glu Cys Pro Trp Gln Val Leu Leu
           195                 200                 205
   Leu Val Asn Gly Ala Gln Leu Cys Gly Gly Thr Leu Ile Asn Thr Ile
       210                 215                 220
   Trp Val Val Ser Ala Ala His Cys Phe Asp Lys Ile Lys Asn Trp Arg
   225                 230                 235                 240
   Asn Leu Ile Ala Val Leu Gly Glu His Asp Leu Ser Glu His Asp Gly
                   245                 250                 255
   Asp Glu Gln Ser Arg Arg Val Ala Gln Val Ile Ile Pro Ser Thr Tyr
               260                 265                 270
   Val Pro Gly Thr Thr Asn His Asp Ile Ala Leu Leu Arg Leu His Gln
           275                 280                 285
   Pro Val Val Leu Thr Asp His Val Val Pro Leu Cys Leu Pro Glu Arg
       290                 295                 300
   Thr Phe Ser Glu Arg Thr Leu Ala Phe Val Arg Phe Ser Leu Val Ser
   305                 310                 315                 320
   Gly Trp Gly Gln Leu Leu Asp Arg Gly Ala Thr Ala Leu Glu Leu Met
                   325                 330                 335
   Val Leu Asn Val Pro Arg Leu Met Thr Gln Asp Cys Leu Gln Gln Ser
               340                 345                 350
   Arg Lys Val Gly Asp Ser Pro Asn Ile Thr Glu Tyr Met Phe Cys Ala
           355                 360                 365
   Gly Tyr Ser Asp Gly Ser Lys Asp Ser Cys Lys Gly Asp Ser Gly Gly
       370                 375                 380
   Pro His Ala Thr His Tyr Arg Gly Thr Trp Tyr Leu Thr Gly Ile Val
   385                 390                 395                 400
   Ser Trp Gly Gln Gly Cys Ala Thr Val Gly His Phe Gly Val Tyr Thr
                   405                 410                 415
   Arg Val Ser Gln Tyr Ile Glu Trp Leu Gln Lys Leu Met Arg Ser Glu
               420                 425                 430
   Pro Arg Pro Gly Val Leu Leu Arg Ala Pro Phe Pro
           435                 440
<210> 5
   <211> 6
   <212> PRT
   <213> Artificial Sequence
<220>
   <223> peptide
<400> 5
   Ala Thr Lys Ala Val Cys
   1 5
<210> 6
   <211> 45
   <212> DNA
   <213> Artificial Sequence
<220>
   <223> FVIIEcoRISS F
<400> 6
   ccggaattca tggccaacgc gttcctggag gagctgcggc cgggc 45
<210> 7
   <211> 62
   <212> DNA
   <213> Artificial Sequence
<220>
   <223> FVII#1XhoIAS R
<400> 7
   ccgctcgagt cagcacacgg ccttcgtcgc gggaaatggg gctcgcagga ggactcctgg 60
gc 62
<210> 8
   <211> 1353
   <212> DNA
   <213> Artificial Sequence
<220>
   <223> FactorVII-ATKAVC DNA
<400> 8
   atggtctccc aggccctcag gctcctctgc cttctgcttg ggcttcaggg ctgcctggct 60
   gcagtcttcg taacccagga ggaagcccac ggcgtcctgc accggcgccg gcgcgccaac 120
   gcgttcctgg aggagctgcg gccgggctcc ctggagaggg agtgcaagga ggagcagtgc 180
   tccttcgagg aggcccggga gatcttcaag gacgcggaga ggacgaagct gttctggatt 240
   tcttacagtg atggggacca gtgtgcctca agtccatgcc agaatggggg ctcctgcaag 300
   gaccagctcc agtcctatat ctgcttctgc ctccctgcct tcgagggccg gaactgtgag 360
   acgcacaagg atgaccagct gatctgtgtg aacgagaacg gcggctgtga gcagtactgc 420
   agtgaccaca cgggcaccaa gcgctcctgt cggtgccacg aggggtactc tctgctggca 480
   gacggggtgt cctgcacacc cacagttgaa tatccatgtg gaaaaatacc tattctagaa 540
   aaaagaaatg ccagcaaacc ccaaggccga attgtggggg gcaaggtgtg ccccaaaggg 600
   gagtgtccat ggcaggtcct gttgttggtg aatggagctc agttgtgtgg ggggaccctg 660
   atcaacacca tctgggtggt ctccgcggcc cactgtttcg acaaaatcaa gaactggagg 720
   aacctgatcg cggtgctggg cgagcacgac ctcagcgagc acgacgggga tgagcagagc 780
   cggcgggtgg cgcaggtcat catccccagc acgtacgtcc cgggcaccac caaccacgac 840
   atcgcgctgc tccgcctgca ccagcccgtg gtcctcactg accatgtggt gcccctctgc 900
   ctgcccgaac ggacgttctc tgagaggacg ctggccttcg tgcgcttctc attggtcagc 960
   ggctggggcc agctgctgga ccgtggcgcc acggccctgg agctcatggt cctcaacgtg 1020
   ccccggctga tgacccagga ctgcctgcag cagtcacgga aggtgggaga ctccccaaat 1080
   atcacggagt acatgttctg tgccggctac tcggatggca gcaaggactc ctgcaagggg 1140
   gacagtggag gcccacatgc cacccactac cggggcacgt ggtacctgac gggcatcgtc 1200
   agctggggcc agggctgcgc aaccgtgggc cactttgggg tgtacaccag ggtctcccag 1260
   tacatcgagt ggctgcaaaa gctcatgcgc tcagagccac gcccaggagt cctcctgcga 1320
   gccccatttc ccgcgacgaa ggccgtgtgc tga 1353
<210> 9
   <211> 450
   <212> PRT
   <213> Artificial Sequence
<220>
   <223> FactorVII-ATKAVC
<400> 9
   Met Val Ser Gln Ala Leu Arg Leu Leu Cys Leu Leu Leu Gly Leu Gln
   1 5 10 15
   Gly Cys Leu Ala Ala Val Phe Val Thr Gln Glu Glu Ala His Gly Val
   20 25 30
   Leu His Arg Arg Arg Arg Ala Asn Ala Phe Leu Glu Glu Leu Arg Pro
   35 40 45
   Gly Ser Leu Glu Arg Glu Cys Lys Glu Glu Gln Cys Ser Phe Glu Glu
   50 55 60
   Ala Arg Glu Ile Phe Lys Asp Ala Glu Arg Thr Lys Leu Phe Trp Ile
   65 70 75 80
   Ser Tyr Ser Asp Gly Asp Gln Cys Ala Ser Ser Pro Cys Gln Asn Gly
                   85                  90                  95
   Gly Ser Cys Lys Asp Gln Leu Gln Ser Tyr Ile Cys Phe Cys Leu Pro
               100                 105                 110
   Ala Phe Glu Gly Arg Asn Cys Glu Thr His Lys Asp Asp Gln Leu Ile
           115                 120                 125
   Cys Val Asn Glu Asn Gly Gly Cys Glu Gln Tyr Cys Ser Asp His Thr
       130                 135                 140
   Gly Thr Lys Arg Ser Cys Arg Cys His Glu Gly Tyr Ser Leu Leu Ala
   145                 150                 155                 160
   Asp Gly Val Ser Cys Thr Pro Thr Val Glu Tyr Pro Cys Gly Lys Ile
                   165                 170                 175
   Pro Ile Leu Glu Lys Arg Asn Ala Ser Lys Pro Gln Gly Arg Ile Val
               180                 185                 190
   Gly Gly Lys Val Cys Pro Lys Gly Glu Cys Pro Trp Gln Val Leu Leu
           195                 200                 205
   Leu Val Asn Gly Ala Gln Leu Cys Gly Gly Thr Leu Ile Asn Thr Ile
       210                 215                 220
   Trp Val Val Ser Ala Ala His Cys Phe Asp Lys Ile Lys Asn Trp Arg
   225                 230                 235                 240
   Asn Leu Ile Ala Val Leu Gly Glu His Asp Leu Ser Glu His Asp Gly
                   245                 250                 255
   Asp Glu Gln Ser Arg Arg Val Ala Gln Val Ile Ile Pro Ser Thr Tyr
               260                 265                 270
   Val Pro Gly Thr Thr Asn His Asp Ile Ala Leu Leu Arg Leu His Gln
           275                 280                 285
   Pro Val Val Leu Thr Asp His Val Val Pro Leu Cys Leu Pro Glu Arg
       290                 295                 300
   Thr Phe Ser Glu Arg Thr Leu Ala Phe Val Arg Phe Ser Leu Val Ser
   305                 310                 315                 320
   Gly Trp Gly Gln Leu Leu Asp Arg Gly Ala Thr Ala Leu Glu Leu Met
                   325                 330                 335
   Val Leu Asn Val Pro Arg Leu Met Thr Gln Asp Cys Leu Gln Gln Ser
               340                 345                 350
   Arg Lys Val Gly Asp Ser Pro Asn Ile Thr Glu Tyr Met Phe Cys Ala
           355                 360                 365
   Gly Tyr Ser Asp Gly Ser Lys Asp Ser Cys Lys Gly Asp Ser Gly Gly
       370                 375                 380
   Pro His Ala Thr His Tyr Arg Gly Thr Trp Tyr Leu Thr Gly Ile Val
   385                 390                 395                 400
   Ser Trp Gly Gln Gly Cys Ala Thr Val Gly His Phe Gly Val Tyr Thr
                   405                 410                 415
   Arg Val Ser Gln Tyr Ile Glu Trp Leu Gln Lys Leu Met Arg Ser Glu
               420                 425                 430
   Pro Arg Pro Gly Val Leu Leu Arg Ala Pro Phe Pro Ala Thr Lys Ala
           435                 440                 445
   Val Cys
       450

## Description

### [Technical Field]

The present invention relates to a method for mass production of human coagulation factor VII derivatives, including: a) constructing an expression vector, containing i) a nucleotide sequence of a dihydrofolate reductase (DHFR, hereinafter) promoter devoid of at least one CCGCCC repeat sequence from the GC-rich region thereof and a nucleotide sequence encoding DHFR operably linked thereto, and ii) a nucleotide sequence of a cytomegalovirus (CMV) early gene promoter and a nucleotide sequence encoding a human coagulation factor VII derivative operably linked thereto; b) transfecting an animal cell line with the expression vector of step a); c) culturing the transfected animal cell line of step b) in the presence of a DHFR inhibitor to select a cell line capable of expressing the human coagulation factor VII derivative with high efficiency; and d) culturing the animal cell line selected from step c) by adding at least one selected from the group consisting of sodium butyrate, vitamin K, and a culture medium supplement; and to a cell line for mass production of human coagulation factor VII derivatives.

### [Background Art]

Human coagulation factor VII (FVII, hereinafter), which is produced in the liver and secreted into the blood, is a precursor of serine protease that causes blood to clot by activating coagulation factor X or coagulation factor IX. FVII is a single-chained glycoprotein with a molecular weight of 50,000 Da. FVII becomes an active form, which is factor VIIa decomposed into two chain forms, by factor Xa, factor XIIa, factor IXa, and thrombin. FVII is known to strengthen enzyme activity by binding to negatively charged phospholipid A (Nemerson et al, Thromb. Res, 1985, 40:351∼358).

In the presence of tissue factors and calcium ions, factor VIIa activates factor X into factor Xa which, in turn, converts prothrombin into thrombin in the presence of factor 5a, calcium ions and phospholipids, thus performing coagulation *in vivo.*

FVII, consisting of a total of 406 amino acids, is activated into factor VIIa by the cleavage between the single peptide bond between arginine (the 152^{nd} amino acid) and isoleucine (the 153^{rd} amino acid). As a result, factor VIIa obtains a structure in which the light chain (consisting of 152 amino acid residues) and the heavy chain (consisting of 254 amino acid residues) are held together by disulfide bonds. The light chain has a gamma carboxyl glutamic acid domain (Gla) and two epidermal growth factor (EGF) domains while the heavy chain has an active site for serine protease.

In order to form 10 carboxyl glutamic acids at the N-terminus, which is involved in biological activity of factor VIIa, vitamin K is required (Hagen et al., Natl. Acad. SC. U.S.A, 1986, 83:2412∼2416). This Gla-domain is known to be involved in binding FVII to the cell surface containing the tissue factor (Sakai, et al., J.Biol Chem, 1990, 265:1890∼1894).

At present, there are two methods for the production of factor VIIa. The first method involves isolating and purifying FVII from plasma and activating FVII into factor VIIa (Broze, et al., J. Biol.Chem, 1980, 225:1242∼1247). The second method involves obtaining factor VIIa by culturing animal cells into which the DNA sequence of FVII was inserted (European Patent Application No: 86302855.1).

The plasma-derived products have low production efficiency and instability of supply, and specifically, they have high risk in terms of safety. In contrast, the genetic recombinant products can overcome the demerits of the plasma-derived products using a genetic engineering technique.

However, the production of FVII in animal cells using a genetic recombinant technique has a problem of low productivity in most cases due to its low expression. Therefore, for the use of FVII as a therapeutic agent, it is necessary to establish a cell line enabling a large scale production. Further, it is essential to develop an expression vector that is expressed with high efficiency for the establishment of the cell line.

### [Disclosure of Invention]

### [Technical Problem]

The present inventors have made extensive efforts to find a method for the mass production of FVII and its derivatives. As a result, they have constructed an expression vector for animal cell lines capable of expressing FVII with high efficiency using a vector with a DHFR promoter devoid of the GC-rich repeat sequence, and have transfected the animal cell lines with the expression vector to construct a monoclonal transfectant capable of stably producing FVII on a large scale. When the present inventors have added sodium butyrate and vitamin K in a concentration ranging from low to high levels and cultured the transfectants to increase the production level of the FVII derivatives from the monoclones, they have confirmed that the expression of FVII derivatives was considerably increased when cultured by adding sodium butyrate and vitamin K at high concentrations, thereby completing the present invention.

### [Technical Solution]

It is an object of the present invention to provide a method for mass production of FVII derivatives, including: a) constructing an expression vector, containing i) a nucleotide sequence of a DHFR promoter devoid of at least one CCGCCC repeat sequence from the GC-rich region thereof and a nucleotide sequence encoding DHFR operably linked thereto, and ii) a nucleotide sequence of a CMV early gene promoter and a nucleotide sequence encoding a FVII derivative operably linked thereto; b) transfecting an animal cell line with the expression vector of step a); c) culturing the transfected animal cell line of step b) in the presence of a DHFR inhibitor to select a cell line capable of expressing the FVII derivative with high efficiency; and d) culturing the animal cell line selected from step c) by adding at least one selected from the group consisting of sodium butyrate, vitamin K, and a culture medium supplement.

It is another object of the present invention to provide an HMF 709 cell line (Accession No. KCTC 12022 BP), which produces FVII derivatives.

It is still another object of the present invention to provide a method for mass production of FVII derivatives, comprising culturing an HMF709 cell line (Accession No. KCTC 12022 BP) capable of producing FVII derivatives by adding at least one selected from the group consisting of sodium butyrate, vitamin K, and a culture medium supplement.

### [Advantageous Effects]

The method of the present invention enables the expression of FVII derivatives with high efficiency on a large scale using a vector containing a DHFR promoter devoid of GC-rich repeat sequences, and thus may be usefully applied to the preparation of therapeutic agents for hemophilia.

### [Brief Description of Drawings]

Fig. 1 shows a map of hFVII-SOD1 (ATKAVC) expression vector.
Fig. 2 shows an expression level of the hFVII-SOD1 (ATKAVC) in colonies obtained from a cell line, which is transformed with the hFVII-SOD1 (ATKAVC) expression vector, measured by ELISA assay.
Fig. 3 shows a doubling time (Td) by applying the colonies obtained from a cell line, which is transformed with the hFVII-SOD1(ATKAVC) expression vector, to a serum-free medium.
Fig. 4 shows the hFVII-SOD1 (ATKAVC), which is expressed in a CHO cell line based on the concentration and temperature of sodium butyrate, as measured by ELISA assay.
Fig. 5 shows the hFVII-SOD1 (ATKAVC), which is expressed in a CHO cell lineaccording to the concentration of vitamin K, as measured by ELISA assay.
Fig. 6 shows a culture profile of a CHO cell line cultured in a bioreactor.
Fig. 7 shows a culture profile of a CHO cell line cultured in the bioreactor using a soy hydrolysate supplement.
Fig. 8 shows a culture profile of a CHO cell line cultured in the bioreactor using a yeast extract supplement.
Fig. 9 shows a culture profile of a CHO cell line cultured in a flask by adding yeast extract at various concentrations.
Fig. 10 shows an expression level of a CHO cell line cultured in the flask by adding the yeast extract at various concentrations.

### [Best Mode for Carrying out the Invention]

In order to achieve the above-described object, in an aspect, the present invention provides a method for mass production of FVII derivatives, including: a) constructing an expression vector, containing i) a nucleotide sequence of a DHFR promoter devoid of at least one CCGCCC repeat sequence from the GC-rich region thereof and a nucleotide sequence encoding DHFR operably linked thereto, and ii) a nucleotide sequence of a CMV early gene promoter and a nucleotide sequence encoding a FVII derivative operably linked thereto; b) transfecting an animal cell line with the expression vector of step a); c) culturing the transfected animal cell line of step b) in the presence of a DHFR inhibitor to select a cell line capable of expressing the FVII derivatives with high efficiency; and d) culturing the animal cell line selected from step c) by adding at least one selected from the group consisting of sodium butyrate, vitamin K, and a culture medium supplement; and a cell line for mass production of FVII derivatives.

Specifically, step a) addresses i) a nucleotide sequence of a DHFR promoter devoid of at least one CCGCCC repeat sequence from the GC-rich region thereof and a nucleotide sequence encoding DHFR operably linked thereto, and ii) a nucleotide sequence of a CMV early gene promoter and a nucleotide sequence encoding a FVII derivative operably linked thereto.

As used herein, the term "GC-rich region" refers to the CCGCCC repeat sequence included in a promoter, which is a transcription regulatory factor of DHFR. When all or a part of the repeat sequence is artificially lost by the method such as deletion or mutation, etc., DHFR expression is achieved at a minimum level. When the DHFR inhibitor is added under the condition in which the expression is maintained at a minimum level, the cells can amplify a higher number of the DHFR genes for survival, and therefore, it was observed that recombinant genes of interest included in the expression vector containing the DHFR genes were also simultaneously amplified and highly expressed.

Therefore, in an exemplary aspect of the present invention, the GC-rich region provides a high expression-inducing cassette including a nucleotide sequence of DHFR genes containing a promoter devoid of at least one CCGCCC repeat sequence.

Preferably, the high expression-inducing cassette contains a DHFR promoter including six or less CCGCCC repeat sequences, more preferably a promoter containing three or less CCGCCC repeat sequences, even more preferably a promoter containing one or less CCGCCC repeat sequence, and most preferably a promoter devoid of CCGCCC repeat sequences.

The removal of these CCGCCC repeat sequences may be achieved by the method including substitution or deletion of the nucleotide sequence according to genetically recombinant techniques widely known in the art. In an exemplary embodiment of the present invention, a part or all of the GC-rich region in the promoter was removed by deleting a part of the nucleotide sequence containing the CCGCCC repeat sequences.

As used herein, the term "DHFR" refers to an enzyme that reduces dihydrofolic acid to tetrahydrofolic acid using NADPH as an electron donor. In humans, DHFR is encoded by a DHFR gene.

As used herein, the term "human coagulation factor" refers to a coagulation-related protein which is involved in body protection by coagulation upon hemorrhage from abrasion. The coagulation is a series of reactions caused by participation of the protein XII.

As used herein, the term "human coagulation factor VII", also known as proconvertin, refers to a heat-labile protein with a molecular weight of 50,000 Da, which is synthesized in the liver and has a blood concentration from 20 mg/mL to 40 mg/mL. FVII may be activated into factor VIIa to be used as a coagulant for coagulation. The nucleotide sequence of factor VII includes the nucleotides, which shares a sequence homology of 70%, 80%, 90%, and 95%, preferably 97%, with the known sequence capable of showing the activity of FVII, without any limitations. The nucleotide sequence may preferably be that represented by SEQ ID NO: 3.

As used herein, the term "human coagulation factor VII derivatives" refers to a substance which can cause deletion, addition, and/or substitution for amino acid or chemical residues, which form the FVII. In particular, these derivatives may have advantageous characteristics that may have been increased or newly added thereto as a therapeutic protein because additional functions, such as an increase of stability *in vivo,* an improvement of storage ability, an increase of activity, and an inclusion of binding sites for other ingredients, etc., have been included through the deletion, addition, and/or substitution for amino acid or chemical residues. In the present invention, the FVII derivatives may be the FVII with a specific amino acid sequence(s) added thereto, and specifically to the C-terminus. The added amino sequence may be a peptide linker. Preferably, part of superoxide dismutase 1 (SOD1, hereinafter) sequence may have been included, and specifically, SEQ ID NOS: 1 to 6 of SOD1 (ATKAVC, SEQ ID NO: 5) may have been included.

As used herein, the term "SOD1 (superoxide dismutase 1)" refers to an enzyme which catalyzes a disproportionation reaction that decomposes superoxide, an oxygen free radical, into oxygen and hydrogen peroxide *in vivo.* SOD1 is known to perform main anti-oxidation defense mechanism in almost all the cells exposed to oxygen. Immunogenicity against additional sequences may be decreased using SOD1-derived sequence, which is a type of protein often found *in vivo.*

As used herein, the term "vector" refers to any vehicle for the cloning and/or transfer of a nucleotide sequence into a host cell. The vector may be a replicon to which another DNA fragment may be conjugated so as to bring about the replication of the conjugated fragment. The term "replicon" refers to any genetic unit (e.g., a plasmid, a phage, a cosmid, a chromosome, a virus, etc.) that functions as an autonomous unit of DNA replication *in vivo,* i.e., one which is capable of replication under its own control. The term "vector" includes both viral and non-viral vehicles for introducing the nucleotide sequence into a host cell *in vitro, ex vivo,* or *in vivo.* The term "vector" may also include minicircle DNAs. For example, the vector may be a plasmid without a bacterial DNA sequence. The removal of the bacterial DNA sequences that are rich in CpG regions has been carried out to decrease transgene expression silencing and bring about more persistent expression from plasmid DNA vectors (Ehrhardt, A. et al. (2003) Hum Gene Ther 10: 215-25; Yet, N. S. (2002) Mol Ther 5: 731-38; Chen, Z. Y. et al. (2004) Gene Ther 11 : 856-64). The term "vector" may also include transposons such as Sleeping Beauty (Izsvak et al. J. Mol. Biol. 302:93-102 (2000)) or artificial chromosomes. In the present invention, the term "expression vector", which may express the protein of interest with high efficiency by expressing the coagulant factors, may be a vector containing i) a nucleotide sequence of a DHFR promoter devoid of at least one CCGCCC repeat sequence from the GC-rich region thereof and a nucleotide sequence encoding DHFR operably linked thereto. For example, the expression vector may include a plasmid vector, a cosmid vector, a bacteriophage vector, and a virus vector, such as an adenovirus vector, a retrovirus vector, an adeno-associated virus vector, etc., and preferably a plasmid vector.

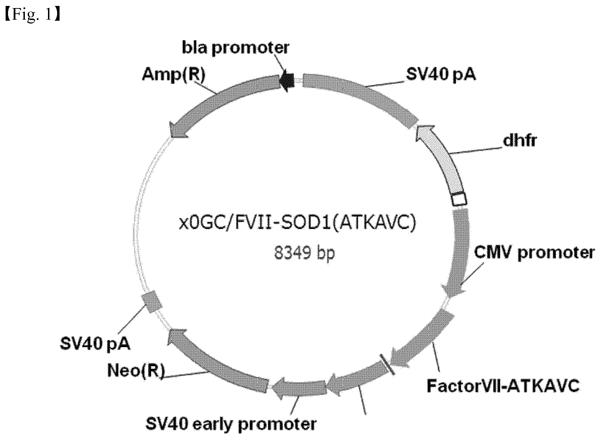

The expression vector may further include a nucleotide sequence encoding FVII derivatives and the FVII derivatives of interest may be expressed with high efficiency by expressing the expression vector. Preferably, the expression vector, which further includes the nucleotide sequence encoding FVII derivatives, may be the pXOGC-FVII-ATKAVC vector described in Fig. 1.

The FVII derivatives may be expressed under the DHFR gene promoter or its expression may be controlled by additional promoters. Preferably, the FVII derivative-expression may be controlled by additional promoters. These promoters, widely known in the art, may be selected from the group consisting of cytomegalovirus (CMV) promoter, LTR promoter, EFα promoter, SV40 promoter and TK promoter, and used by one of ordinary skill in the art, but are not limited thereto.

The expression vector of the present invention, which is for inducing high expression in animal cell lines, may preferably further include a resistance gene for animal cells, which is used as a selectable marker for permanent expression in animal cells. The resistance genes for the animal cells include those conventionally used in the art, such as a neomycin resistance gene, a zeomycin resistance gene, a hygromycin resistance gene, a blastomycin resistance gene, etc., but are not limited thereto.

Additionally, the expression vector of the present invention may further include general constituent elements of a vector, such as a replication origin and a polyadenylation signal, other transcriptional control elements, etc., but is not limited thereto.

Preferably, step (b) of the present invention relates to the transfection of an animal cell line with the expression vector of step a).

As used herein, the term "transformation" or "transfection" refers to any artificial genetic alteration resulting from the introduction of a foreign gene into a host cell for the self-replication of the introduced gene or an insertion into the host genome.

A method for transforming the vector of the present invention within a cell may include any method for introducing a nucleotide sequence into the cell and may be carried out by selecting a suitable standard technique known in the art. The technique may include electroporation, calcium phosphate co-precipitation, retroviral infection, microinjection, DEAE-dextran, cationic liposome calcium, etc., but are not limited thereto.

Specifically, in the present invention, a vector expressing a recombinant protein is transformed into a CHO cell with the aid of lipofectamine.

Further, in the present invention, culturing of the animal cell line may be performed in a suitable culture medium culture conditions known in the art. The culture process may be readily adjusted and used based on the selected animal cell lines by one of ordinary skill in the art. The culture process may be classified into a suspension culture and an attachment culture according to the growth type of cells and into a batch-type method, a fed-batch type method, and a continuous type method according to the culture method. The culture medium used for culture must adequately meet the requirement for specific cell lines.

For culturing animal cells, the culture medium may contain various ingredients such as a carbon source, a nitrogen source, and trace elements. Examples of the usable carbon sources may include carbohydrates, such as glucose, sucrose, lactose, fructose, maltose, starch, and cellulose; fats, such as soybean oil, sunflower oil, castor oil, and coconut oil; fatty acids, such as palmitic acid, stearic acid, and linoleic acid; alcohols, such as glycerol and ethanol; and organic acids, such as acetic acid. These carbon sources may be used alone or in combination. Examples of the usable nitrogen sources may include an organic nitrogen source, such as peptone, yeast extract, broth, malt extract, corn steep liquor (CSL), and soybean; and an inorganic nitrogen source, such as urea, ammonium sulfate, ammonium chloride, ammonium phosphate, ammonium carbonate, and ammonium nitrate. These nitrogen sources may be used alone or in combination. Besides, amino acids, vitamins, suitable precursors, etc., may be further included.

Additionally, the culture medium may be added with a DHFR inhibitor, such as methotrexate. That is, as described above, because the protein recombination method according to an exemplary embodiment of the present invention aims to effectively establish in a short period of time a system by which DHFR in a vector can be amplified and selected by transfecting a DHFR-deficient animal cells with an expression vector according to the present invention and adding a DHFR inhibitor so as to amplify a recombinant gene.

The recombinant protein of interest requires its expression in animal cell lines. For this purpose, examples of preferable animal cells used in the present invention may include a Chinese hamster ovarian (CHO) cancer cell line, a monkey kidney cell line (COS7), an NSO cell line, an SP2/0 cell line, a W138 cell line, a baby hamster kidney (BHK) cell line, MDCK cells, a myeloma cell line, an HuT 78 cell line, a 293 cell line, etc., but are not limited thereto. One of ordinary skill in the art may readily select an animal cell line suitable for application in the amplification technique using DHFR of the present invention. Preferably, in the present invention, the cell line may be deficient in the DHFR gene.

As used herein, the term "host cell transformed with a recombinant vector", which refers to a cell transfected with a vector containing genes encoding at least one protein of interest, may be recombinant mammalian cells, rodent cells, preferably animal cells or animal-derived cells, and most preferably CHO cells. Because the purpose is to stably express the gene and to amplify the copy number of the gene in the cell when the host cell is the CHO cell, a vector (e.g., pCHOI, etc.) having a DHFR gene that is complementary to that of the CHO cell may be introduced into the CHO cell deficient in the nucleotide synthesis pathway, and amplified with methotrexate (MTX, hereinafter).

In an exemplary embodiment of the present invention, a DHFR-deficient Chinese hamster ovarian cell line (CHO/dhfr-) was used. That is, DHFR-deficient CHO cells were transfected with an expression vector containing a gene encoding FVII according to the present invention. In the transformed CHO cells, a sufficient amount of the gene was amplified and the animal cell lines with verified productivity was provided even at an MTX concentration of 100 nM or less, and more preferably at 50 nM or less.

Preferably, step c) of the present invention relates to the culturing of the transfected animal cell line of step b) in the presence of a DHFR inhibitor to select a cell line capable of expressing the FVII derivative with high efficiency.

Preferably, the cell line selected from step c) may be HMF709 (KCTC 12022BP).

In an exemplary embodiment of the present invention, the selected cell line was named as HMF709, and the HMF709 cell line was deposited in the Korean Collection for Type Culture at the Korean Research Institute of Bioscience and Biotechnology (Korean Research Institute of Bioscience and Biotechnology, 111 Gwahak-ro, Yuseong-gu, Daejeon, Korea), an international depository authority under Budapest Treaty, on September 23, 2011, under Accession No. KCTC 12022BP.

On the other hand, the cell line selected from step c) may be applied to a suspension culture using a serum-free medium (EX-CELL CHO culture medium, Sigma, USA, Cat. No. 14360C). In an exemplary embodiment of the present invention, HMF 709 (KCTC12022BP) was applied to a suspension culture using a serum-free medium (EX-CELL CHO culture medium, Sigma, USA, Cat. No. 14360C).

Further, in the present invention, step d) relates to the culturing of the animal cell line selected from step c) by adding at least one selected from the group consisting of sodium butyrate, vitamin K, and a culture medium supplement.

As used herein, the term "sodium butyrate", which is known as a substance causing histone hyperacetylation by inhibition of histone deacetylase, is known to induce cell differentiation and gene expression. In the present invention, sodium butyrate was used as a culture medium supplement for mass production of FVII derivatives. The amount thereof may be readily selected by one of ordinary skill in the art based on the culture conditions and may be added preferably in a concentration from 0.1 mM to 4.0 mM, more preferably in a concentration from 1.1 mM to 3.5 mM when the culture temperature of step d) is from 32.5°C to 35.0°C, and in a concentration from 1.2 mM to 2.0 mM when the culture temperature is from 31°C to 32.5°C. Specifically, when the culture temperature of step d) is 33.0°C, sodium butyrate may be added in a concentration of 1.5 mM.

The FVII derivatives may be expressed under the DHFR gene promoter or their expression may be controlled by additional promoters. Preferably, the FVII derivative-expression is controlled by additional promoters. These promoters, widely known in the art, may be selected from the group consisting of CMV promoter, LTR promoter, EFα promoter, SV40 promoter, and TK promoter, and used by one of ordinary skill in the art, but are not limited thereto.

As used herein, the term "vitamin K", which generally refers to all of vitamin K1 (phylloquinone), K2 (menaquinone), and the precursor K3 (menadione), is a type of fat-soluble vitamin that stimulates coagulation. The "K" in vitamin K is derived from the German word "koagulation", which means coagulation. Specifically, vitamin K is known to assist normal coagulation by being involved in the formation of coagulation protein such as prothrombin. Further, vitamin K is known to play a role in carboxylation of osteocalcin protein, which is mainly present in bones. In the present invention, vitamin K may be vitamin K1 or K3, specifically K1, but is not limited thereto.

As used herein, the term "culture medium supplement" includes all the ingredients that may be added to a culture medium capable of culturing cells and subjects, and specifically, may stimulate cell survival and cell proliferation of animal cell lines or protein synthesis, but they are not limited thereto. In the present invention, the culture medium supplement may specifically be soy hydrolysate or yeast extract. The soy hydrolysate or yeast extract in a concentration from 0.1 g/L to 3 g/L may be added to the culture medium.

In an exemplary embodiment of the present invention, two inducible high-expression cassettes, in which one contains only one CCGCCC repeat sequence from the DHFR promoter and the other contains none at all, were prepared, respectively. *E. coli* cell lines, which were transformed using the expression vector containing these cassettes, were used. The cell lines were deposited in the gene bank at the Korean Research Institute of Bioscience and Biotechnology located in Yuseong-gu, Daejeon, Korea on October 2, 2006 under Accession Nos. KCTC 10991 BP and KCTC 10992 BP, respectively, in the prior invention by the present inventors. In order to induce high expression of a recombinant protein of interest, the cell lines may be used for the construction of an expression vector, which further includes the genes encoding FVII derivative protein by isolating the expression vector containing the high-expression inducible cassettes from the cell line using a cloning method such as genetic recombinant technique. The pXOGC-FVII-SOD1 (ATKAVC) vector illustrated in Fig.1 was prepared as the expression vector to be used in the present invention. The CHO cell line transformed with the expression vector was cultured in a culture medium supplemented with sodium butyrate, vitamin K or culture medium supplement (soy hydrolysate or yeast extract) to produce FVII derivatives on a large scale.

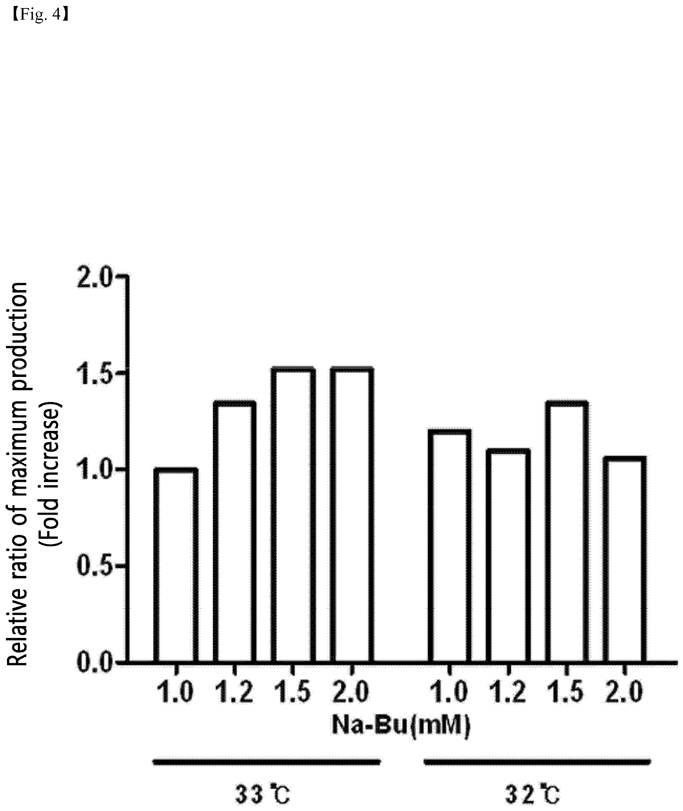

First, as confirmed in Fig. 4, the addition of sodium butyrate increased the production level of hFVII-SOD1 (ATKAVC) specifically at a sodium butyrate concentration of 1.5 mM at 33°Cd.

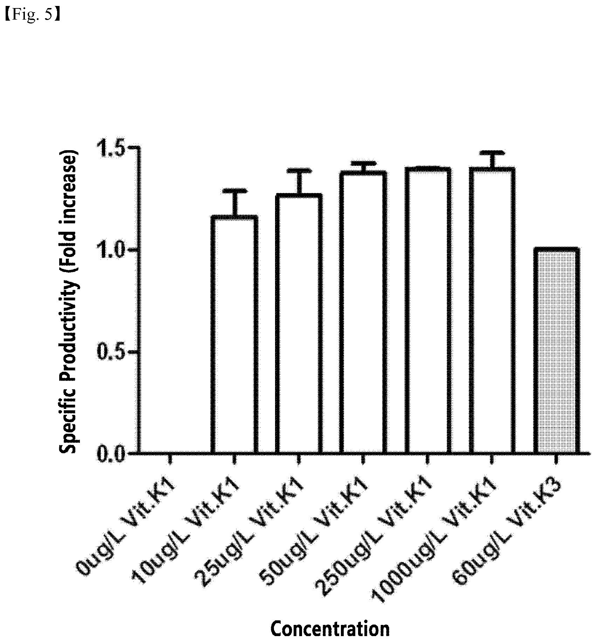

Further, as confirmed in Fig. 5, the increase in the amount of vitamin K1 addition, with a predetermined amount of vitamin K3 set as a control, increased the expression of FVII derivatives in a concentration-dependent manner without any cytotoxicity, even after a high amount of addition. That is, the amount of hFVII-SOD1 (ATKAVC) production was shown to increase along with the increase of vitamin K1 concentration being added thereto.

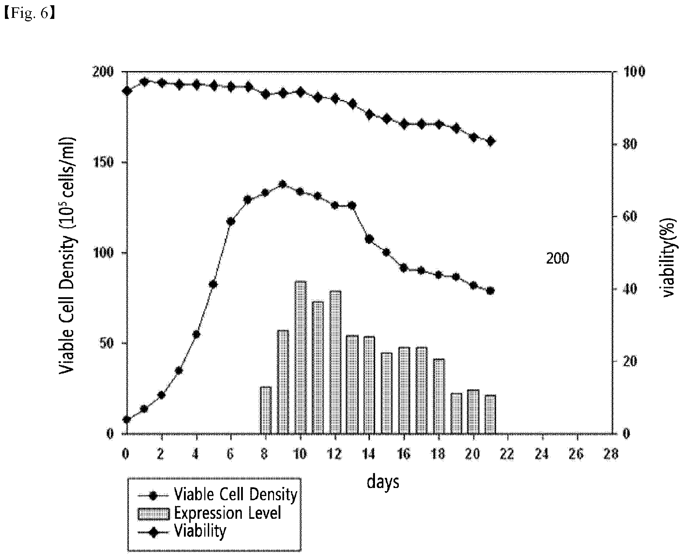

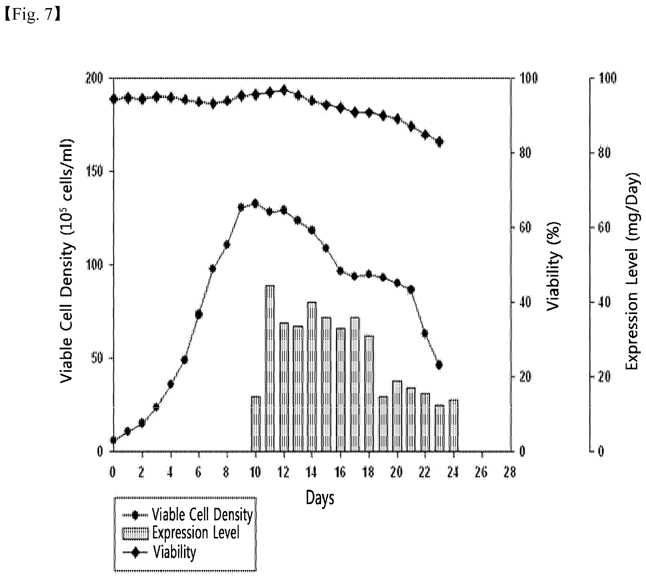

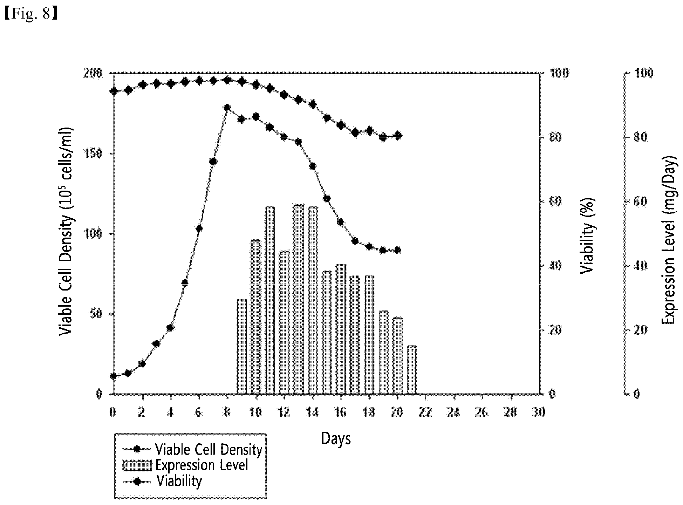

Finally, as can be seen in Table 3 and Figs. 6 to 8, when sodium butyrate or a culture medium supplement (soy hydrolysate or yeast extract) was used as a control, the production of hFVII-SOD1 (ATKAVC) was increased when 1 g/L of soy hydrolysate and 1 g/L of yeast extract were added, respectively. Specifically, a higher production increase was observed when soy hydrolysate was added.

Further, when producing FVII derivatives in the aforementioned cell line, the present invention may further include a large-scale purification of the produced FVII derivatives, in addition to the addition of sodium butyrate. Further, the present invention may further include activation of the produced FVII derivatives.

In an exemplary embodiment of the present invention, the GC-rich sequences of the DHFR promoter were artificially deleted to minimize the DHFR expression and a DHFR inhibitor was added to amplify the introduced gene. Monoclonal cell lines were obtained by limiting dilution from a single cell among the cell lines with gene amplification. The monoclonal cell lines thus obtained were cultured in a serum-free medium on a large scale by adding sodium butyrate, vitamin K, or a culture medium supplement to produce FVII derivatives. Additionally, purified and activated FVII derivatives were produced.

Additionally, the present invention provides a method for mass production of FVII derivatives, including culturing the HMF709 cell line (Accession No. KCTC 12022 BP) capable of producing FVII derivatives by adding at least one selected from the group consisting of sodium butyrate, vitamin K, and a culture medium supplement.

In another aspect, the present invention provides a cell line for producing FVII derivatives. The cell line of the present invention may preferably be the HMF709 cell line (KCTC 12022BP).

In an exemplary embodiment of the present invention, cell lines, which were transformed with the hFVII-SOD1 (ATKAVC) expression vector and cultured in the presence of a DHFR inhibitor to express FVII derivatives with high efficiency, was selected. Then, the cell lines with the highest expression ability for FVII derivatives were selected and deposited in the Korean Collection for Type Culture at the Korean Research Institute of Bioscience and Biotechnology (Korean Research Institute of Bioscience and Biotechnology, 111 Gwahak-ro, Yuseong-gu, Daejeon, Korea) on September 23, 2011, under Accession No. KCTC 12022BP.

### [Mode for Carrying out the Invention]

Hereinafter, the present invention will be described in details with reference to the following Examples. However, these Examples are for illustrative purposes only, and the scope of the present invention is not limited to these Examples.

### Example 1: Construction of expression vector (pX0GC-hFVII) for recombinant FVII expression

### 1-1. Obtaining human FVII genes

First, a human FVII gene containing a signal sequence was obtained by polymerase chain reaction (PCR). For the amplification of the FVII gene, PCR was performed using a human fetal liver cDNA library (TAKARA BIO Inc., USA) as a template, along with the forward and reverse primers of SEQ ID NOS: 1 and 2 shown below.

After denaturing a reaction mixture containing a polymerase, the primers, the cDNA library, and dNTP at 95°C for 1 min, 30 cycles of a reaction(at 95°C for 30 sec, 60°C for 30 sec, and 68°C for 90 sec) were performed followed by a reaction at 68°C for 5 min.

In particular, for the convenience of cloning, a BamHI recognition site was inserted into the primer represented by SEQ ID NO: 1 and a XhoI recognition site was inserted into the primer represented by SEQ ID NO: 2. The primers are shown in Table 1.

**[Table 1] Primers for Amplifying Human FVII Genes**

| Factor VII Primer | Nucleotide Sequence | SEQ ID NO |
|---|---|---|
| Forward Primer (VIIBHISS) | cccggatccatggtctcccaggccctcaggctcc | 1 |
| Reverse Primer (VIIXhoIAS) | gggctcgagctagggaaatggggctcgcagg | 2 |

The nucleotide sequence of the PCR product, which had a size of about 1.3 kb obtained from PCR, was confirmed by DNA sequencing and shown to have the nucleotide sequence represented by SEQ ID NO: 3.

### 1-2. Construction of recombinant FVII expression vector (pX0GC-FVII)

For the expression under the control of a CMV promoter, the PCR product of FVII obtained in Example 1-1 was cloned into an animal cell expression vector, pX0GC vector. The pX0GC vector, which is an expression vector containing a nucleotide sequence of a DHFR promoter devoid of at least one CCGCCC repeat sequence and a nucleotide sequence encoding a DHFR operably linked thereto, is a vector prepared for inducinghigh expression of recombinant proteins (Korean Patent No. 880509).

Specifically, the 1.3 kb-long FVII gene obtained by PCR was digested by incubating at 37°C for 2 hours with restriction enzymes, BamHI and XhoI, and applied to a PCR purification kit (Qiagen, USA), and the digested DNA fragments were obtained. Further, the animal cell expression vector, pX0GC vector, was also digested with the same restriction enzymes, i.e., BamHI and XhoI, under the same conditions as described above, and separated and purified by electrophoresis. The digested DNA fragments and pX0GC vector were mixed and cloned using T4 DNA ligase, thereby preparing an expression vector (pX0GC-FVII) containing FVII genes.

### Example 2: Construction of expression vector for recombinant FVII derivatives

Polynucleotides encoding FVII derivatives, in which part of SOD1 is conjugated to the C-terminus of FVII, were obtained using the expression vector (pX0GC-FVII) containing FVII genes, and an expression vector capable of expressing the derivatives was constructed.

### 2-1. Obtaining human FVII derivative genes

An expression vector (pX0GC-FVII-ATKAVC) for expressing recombinant FVII derivatives, which contains polynucleotides encoding SOD1 SEQ ID NOS: 1 to 6 (ATKAVC, SEQ ID NO: 5) at the 3'-terminus of the FVII genes that were added into the expression vector pX0GC-FVII prepared in Example 1, was constructed. Specifically, a PCR reaction (denaturation at 95°C for 1 min; 30 cycles of a reaction (at 95°C for 60 sec, 60°C for 60 sec, and 68°C for 90 sec), followed by a reaction at 68°C for 5 min) was performed using the expression vector, pX0GC-FVII, as a template, along with the forward and reverse primers represented by SEQ ID NOS: 6 and 7 shown below. In particular, for the convenience of cloning, EcoRI recognition site was inserted into the primer of SEQ ID NO: 6 and XhoI restriction recognition site was inserted into the primer of SEQ ID NO: 7. Then, the nucleotide sequence of the about 1.4 kb-long PCR product obtained from the above PCR reaction (SEQ ID NO: 8) was identified.

**[Table 2]**

| Primers for Amplifying Human FVII Genes | | |
|---|---|---|
| Factor VII Primer | Nucleotide Sequence | SEQ ID NO |
| Forward Primer (FVIIEcoRISS F) | ccggaattcatggccaacgcgttcctggaggagctgcggccgggc | 6 |
| Reverse Primer (FVII#1XhoIAS) | | 7 |

### 2-2. Construction of recombinant VII derivative-expression vector (pX0GC-FVII)

For the expression under the control of a CMV promoter, the PCR product of FVII derivatives obtained above was cloned into an animal cell expression vector, pX0GC vector. Specifically, the PCR product was digested by incubating at 37°C for 2 hours with the restriction enzymes, EcoRI and XhoI, and the digested DNA fragments were obtained using a PCR purification kit (Qiagen, USA). Further, the animal cell expression vector, pX0GC vector, was also digested with the same restriction enzymes, i.e., EcoRI and XhoI, under the same conditions as described above, and separated and purified by electrophoresis. The digested DNA fragments and pX0GC vector were mixed and cloned using T4 DNA ligase, thereby preparing an expression vector (pX0GC-FVII-ATKAVC) containing FVII genes.

### Example 3: Construction of cell lines expressing human FVII derivatives (hFVII-SOD1 (ATKAVC))

The hFVII derivatives were expressed using the expression vector constructed in Example 2.

### 3-1. Transformation of FVII derivatives (pX0GC-FVII-ATKAVC) using a CHO cell line

The recombinant expression vector, pX0GC-FVII-ATKAVC, prepared in Example 2-2 was introduced into DG44/CHO cell line (CHO/dhfr-) (Urlaub et al., Somat. Cell. Mol. Genet., 12, 555-566, 1986), in which the nucleic acid biosynthesis process is incomplete due to DHFR gene damage, to obtain transformants, and FVII-ATKAVC derivatives were expressed in the transfectants.

Specifically, the DG44/CHO cell line was cultured enough to cover 80% to 90% of the bottom of the culture container, and then, the cells were washed three times with Opti-MEM (Gibco, Cat. No. 51985034).

Meanwhile, a mixture of 3 mL of Opti-MEM and 5 µg of the expression vector (pX0GC-FVII-ATKAVC) and a mixture of 3 mL of Opti-MEM and 20 µL of lipofectamin (Gibco, Cat. No. 18324-012) were placed at room temperature for 30 minutes, respectively. Then, each of the mixtures was mixed, added to the cultured DG44/CHO cell line, and cultured under the condition of 5% CO₂ at 37°C for about 18 hours, thereby introducing the expression vector, pX0GC-FVII-ATKAVC, into the DG44/CHO cell line. Subsequently, the cultured cells were washed three times with DMEM-F12 culture medium (Gibco, Cat. No. 11330) containing 10% FBS, added with the culture medium, and cultured again for 48 hours. The cultured cells were isolated individually by adding trypsin thereto, and inoculated to MEM-α culture medium (Welgene, CatNo. LM008-02) containing 10% FBS and 1 mg/mL of G418 (Cellgro, Cat No. 61-234-RG) but without hypoxanthine-thymidine (HT) supplement. Until only the transformed cells were survived to form colonies, the culturing was continued while replacing the selective culture medium every 2 to 3 days to select the transformed cells from the isolated cells. In particular, for improving the expression level of FVII-ATKAVC derivatives in the selected transformed cells, concentration was gradually increased by adding 10 nM of MTX (Sigma, Cat. No. M8407) to the selective culture medium, and the MTX content was increased to 30 nM after 2 to 3 weeks.

Furthermore, for reducing the heterogeneity of the transformed cells, monoclones were obtained the limiting dilution method. Specifically, the transformed cells, which were diluted at a ratio of 0.7 cell/well, were aliquoted into 96-well plates, respectively, and cultured for 2 to 3 weeks to confirm the appearance of monoclones. Upon detection of the monoclones, they were transferred to 24-well plates, and the cell growth rate and the expression level of FVII derivatives for each clone were analyzed by ELISA assay to select the clone with the highest expression level of FVII derivatives.The clone was named as "HMF709", and the corresponding cell line was deposited in the Korean Collection for Type Culture at the Korean Research Institute of Bioscience and Biotechnology located at 111 Gwahak-ro, Yuseong-gu, Daejeon, Korea, an international depository authority under Budapest Treaty, on September 23, 2011, under Accession No. KCTC 12022BP.

### 3-2. Confirmation of hFVII-SOD1 (ATKAVC) expression by ELISA assay

Part of the cells, which were tranfected in Example 3-1, were transferred to 24-well plates in a concentration of 2×10⁴ cells/well and cultured enough to cover almost all the bottom of the culture container. Then, a serum-free medium, CHO-A-SFM (Gibco, Cat. No. 05-5072EF), containing 0.3 mM of sodium butyrate (Sigma, Cat. No. B5887), was placed in an amount of 200 µL per each well, and cultured in a 5% CO₂ incubator at 33°C for 48 hours. The culture medium of the cells was transferred to a 1.5 mL-tube and centrifuged. Subsequently, the supernatants were combined and the expression level of hFVII-SOD1 (ATKAVC) was measured. The measurement of expression level was performed using the ELISA kit (Molecular Innovation, Cat. No. HFVII KT-TOT) based on the manufacturer's protocol.

Specifically, first, the culture broth and the standard substance contained in the kit were diluted with Tris Buffer (0.1 M Tris, 0.15 M NaCl, pH 7.4) at a predetermined ratio, added to the wells of the kit in an amount of 100 µL/well, and reacted in a plate shaker at room temperature at 300 rpm for 30 minutes. Then, the wells were washed four times using the manufacturer's washing solution, and anti-hFVII antibody was added to the wells in an amount of 100 µL/well in a plate shaker at room temperature at 300 rpm for 30 minutes. The wells were washed again four times with the manufacturer's washing solution, and the antibody for horseradish peroxidase (HRP)-conjugated anti-hFVII antibody was added to each well in an amount of 100 µL, and the wells were reacted at 300 rpm for 30 minutes in the same manner described above. After the reaction, the wells were washed four times and reacted at room temperature while adding 100 µL of the substrate to each well. About five minutes later, 50 µL of stopping solution was added to stop the reaction and the absorbance was measured at 450 nm. A standard curve and a function were obtained from the concentrations of the standard solution and the absorbance values provided by the kit manufacturing company. Then, the expression level of hFVII-SOD1 (ATKAVC) was quantified using the standard curve and the function. As a result, it was confirmed that the cells, which were selected and transformed, expressed a certain amount of hFVII-SOD1 (ATKAVC).

### 3-3. Selection of hFVII-SOD1 (ATKAVC)-expressing cell lines

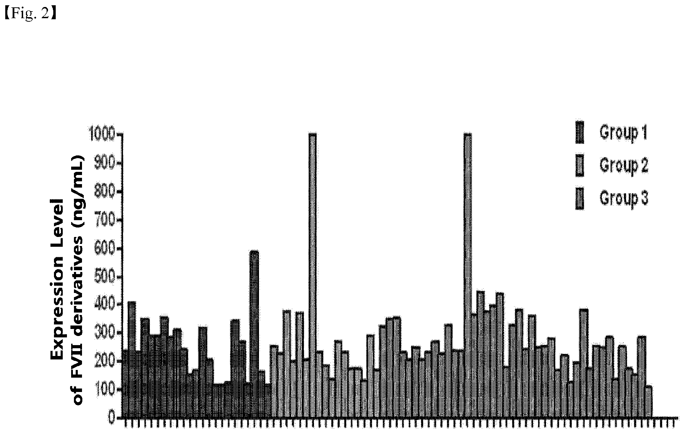

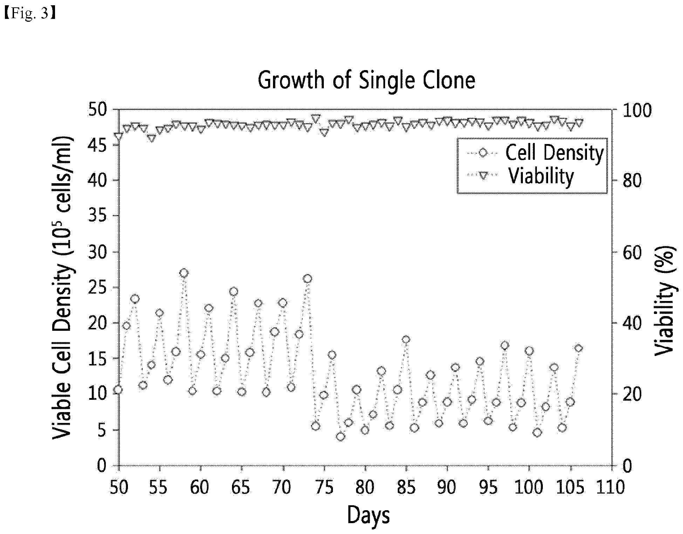

A monoclone selection was performed in a mixed population, in which the hFVII derivative expression was confirmed in Example 3-2 using the limiting dilution method. That is, an experiment for separating monoclines, which had homogeneous expression and high productivity of hFVII derivatives, was performed according to the limiting dilution method using the clones showing a heterogeneous hFVII derivative expression level. Specifically, the cells in the well, which showed the highest expression level among the cells of 6-well plates, were diluted to be inoculated in a density of 1 or less cell per well. Then, the plate in which only a sinlge cell was inoculated was selected and cultured for 2 to 3 weeks. Subsequently, the colony-forming cells were separated from the well plates and subcultured, and the expression level of hFVII derivatives was measured by ELISA assay (Fig. 2). Among the cell lines separated by the limiting dilution method, a recombinant CHO cell line exhibiting high productivity of hFVII derivatives was finally selected and named as "HMF 709". The selected cell line, "HMF709", was deposited in the Korean Collection for Type Culture at the Korean Research Institute of Bioscience and Biotechnology located at 111 Gwahak-ro, Yuseong-gu, Daejeon, Korea) on September 23, 2011, under Accession No. KCTC 12022BP. The selected cell lines were applied to suspension culture using a serum-free medium (EX-CELL CHO culture medium, Sigma, Cat. No. 14360C, USA) (Fig. 3).

### Example 4: Measurement of growth of a cell line and production level of hFVII-SOD1 (ATKAVC) according to the addition of sodium butyrate

### 4-1. Seed and main culture

One vial (1×10⁷ cells/mL) of the hFVII-expressing cell line, which was selected in Example 3-3, subjected to suspension culture, and stored in a liquid nitrogen tank, was taken and thawed as soon as possible in a 37°C constant temperature water bath. Then, the cell line was washed once with a seed culture medium (EX-CELL CHO culture medium (Sigma, Cat. No. 63225C) added with 0.3 g/L of glutamine), centrifuged at 90 ×g for 5 min, and then inoculated into an Erlenmeyer flask (Corning, Cat. No. 431144, USA) containing 50 mL of the seed culture medium. After culturing for 1 to 2 days to a cell concentration of 10×10⁵ cell/mL in a CO₂ incubator (37 °C, 5% CO₂), the cells were centrifuged again in the same manner as described above, and subcultured in a fresh Erlenmeyer flask containing 100 mL of fresh seed culture medium. The subculturing was performed in the same manner while continuously increasing the culture volume by a two-fold subcultureduntil a sufficient number of cells were obtained.

### 4-2. Measurement of the growth of a cell line and the production level of hFVII-SOD1 (ATKAVC) according to the addition of sodium butyrate in the production medium

In order to examine the effect of sodium butyrate addition in the production medium on hFVIIs-SOD1(ATKAVC) production level, about 5.0×10⁶ cells/mL of recombinant CHO cell lines, which were under cultivation, were inoculated into three Erlenmeyer flasks in a culture volume of 50 mL. In particular, 1.0 mM, 1.2 mM, 1.5 mM, and 2.0 mM of sodium butyrate (SIGMA, Cat. No. B5887, USA) was added to the culture medium, respectively, and the culturing started with the flask containing 1.0 mM of sodium butyrate as a control. For the production of hFVII-SOD1 (ATKAVC) n, the cell line was cultured at 32.0°C or 33.0°C for 3 days. On day 3 of culturing, the culture broth was collected and the cell concentration and the level of cell activity of the cell line were examined. As a result, it was confirmed that there were no changes in the cell concentration and the level of cell activity when sodium butyrate was added.

On the other hand, on day 3 of culturing, the supernatant was collected by centrifugation, and hFVII-SOD1 (ATKAVC) expressed in each supernatant was measured by ELISA assay, as disclosed in Example 3-2. The measured values were expressed as the percentage of the production level compared to that of the control (Fig. 4).

As indicated in Fig. 4, specifically, it was confirmed that the production level was highest when 1.5 mM of sodium butyrate was added at 33.0°C.

### Example 5: Measurement of hFVII-SOD1 (ATKAVC) production level according to vitamin K concentration

### 5-1. Seed and main culture

One vial (1×10⁷ cells/mL) of the hFVII-SOD1 (ATKAVC)-expressing cell line, which was selected in Example 3-3, subjected to suspension culture, and stored in a liquid nitrogen tank, was taken and thawed as soon as possible in a 37°C constant temperature water bath. Then, the cell line was washed once with a seed culture medium (EX-CELL CHO culture medium (Sigma, Cat. No. 63225C) added with 0.3 g/L glutamine), centrifuged at 90 ×g for 5 min, and then inoculated into an Erlenmeyer flask (Corning, Cat. No. 431144, USA) containing 50 mL of the seed culture medium. When grown for 1 day to 2 days to a cell concentration of 10×10⁵ cell/mL in a CO₂ incubator (37 °C, 5% CO₂), the cells were centrifuged again in the same manner as described above, and subcultured in a fresh Erlenmeyer flask containing 100 mL of a fresh seed culture medium. The subculturing was performed in the same manner while continuously increasing the culture volume by two-fold subcultureduntil a sufficient number of cells were obtained.

### 5-2. Measurement of the growth of a cell line and the production level of hFVII-SOD1 (ATKAVC) according to the addition of vitamin K in the production medium

In order to examine the effect of vitamin K addition in the production medium on hFVII-SOD1 (ATKAVC) production level, about 5.0×10⁶ cells/mL of recombinant CHO cell lines, which were under cultivation, were inoculated into 3 Erlenmeyer flasks in a culture volume of 50 mL. In particular, 0 µg/L, 10 µg/L, 25 µg/L, 50 µg/L, 250 µg/L, and 1000 µg/L of vitamin K (Merck Millipore, Cat. No. 501890, USA) was added to the culture medium, and culturing started with the flask containing 60 µg/L of vitamin K3 (SIGMA, Cat. No. M2518, USA) as a control. For hFVII-SOD1 (ATKAVC) production, the cell line was cultured at temperature of 33.0°C for 3 days. On day 3 of culturing, the culture fluid was collected and the cell concentration and the level of cell activity of the cell line was observed. As a result, there were no changes in the cell concentration and cell activity level according to vitamin K.

On the other hand, on day 3 of culturing, the supernatant was collected by centrifugation, and then, hFVII-SOD1 (ATKAVC) expressed in each supernatant was measured by ELISA assay, as disclosed in Example 3-2. The measured values were expressed as a production level (multiple) compared to that of the control (Fig. 5).

As indicated in Fig. 5, it was observed that hFVII-SOD1 (ATKAVC) production level increased as the concentration of vitamin K addition increased.

### Example 6: Measurement of growth of a cell line and the production level and activity of hFVII-SOD1(ATKAVC) according to the use of culture medium additive

### 6-1. Seed and main culture

One vial (1×10⁷ cells/mL) of the hFVII-SOD1 (ATKAVC)-expressing cell line, which was selected in Example 3-3, subjected to suspension culture, and stored in a liquid nitrogen tank, was taken and thawed as soon as possible in a 37°C constant temperature water bath. After that, the cell line was washed once with a seed culture medium (EX-CELL CHO culture medium (Sigma, Cat. No. 63225C) supplemented with 0.3 g/L glutamine), centrifuged at 90 ×g for 5 min, and then inoculated into an Erlenmeyer flask (Corning, Cat. No. 431144, USA) containing 50 mL of the seed culture medium. When grown for 1 to 2 days to a cell concentration of 10×10⁵ cell/mL in a CO₂ incubator (37 °C, 5% CO₂), the cell lines were centrifuged again in the same manner as described above, and subcultured in a fresh Erlenmeyer flask containing 100 mL of a fresh seed culture medium. The subculturing was performed in the same manner while continuously increasing the culture volume by a two-fold subcultureduntil a sufficient number of cells were obtained.

### 6-2: Measurement of the growth of a cell line and the production level of hFVII-SOD1 (ATKAVC) by the use of an additive in a production medium

In order to examine the effect of supplement use in the production medium on the hFVII-SOD1(ATKAVC) production level, about 4.0 × 10⁶ cells/mL of recombinant CHO cell lines, which were under cultivation, were inoculated into a 5L-bioreactor in a culture volume of 4 L. When the desired cell concentration of 15 x 10⁶ cells/mL was reached, 1.0 mM of sodium butyrate (SIGMA, Cat. No. B5887, USA), 1 g/L of sodium hydrolysate (SIGMA, Cat. No. 58903C, USA) or 1g/L of yeast extract (SIGMA, Cat. No. Y4375, USA) were added to the culture medium, and the culturing started with a bioreactor containing 1.0 mM of sodium butyrate alone as a control. For hFVII-SOD1 (ATKAVC) production, the cell line was cultured at 33.0°C for at least 10 days. The supernatant was collected on a daily basis and the cell concentration and the level of cellu activity of the cell line were observed.

On the other hand, the supernatant was collected by centrifugation, and then, hFVII-SOD1 (ATKAVC) expressed in each supernatant was measured by ELISA assay, as disclosed in Example 3-2. The culture profile and measured value of the bioreactor were calculated and expressed (Figs. 6, 7, and 8).

**[Table 3]**

| hFVII-SOD1 (ATKAVC) production level (%) based on the use of additive | |
|---|---|
| Additives | hFVII-SOD1(ATKAVC) production level (% compared to control) |
| Control | 100.0 |
| Soy hydrolysate (1g/L) | 113.8 |
| Yeast extract (1g/L) | 111.7 |

As indicated in Table 3 above, it was observed that the hFVII-SOD1 (ATKAVC) production level increased when 1g/L of soy hydrolysate and 1g/L of yeast extract were added. Specifically, it was confirmed that the production level increased highest when soy hydrolysate was addedwhen adding soy hydrolysate.

### 6-3: hFVII-SOD1(ATKAVC) potency analysis based on the use of an additive in production medium

In order to examine the effect of the use of an additive in the production medium on the potency of hFVII-SOD1(ATKAVC), about 4.0 × 10⁶ cells/mL of recombinant CHO cell lines, which were under cultivation, were inoculated into a 5L-bioreactor in a culture volume of 4 L. When the desired cell concentration of 15 x 10⁶ cells/mL was reached, 1.0 mM of sodium butyrate (SIGMA, Cat. No. B5887, USA), 1 g/L of sodium hydrolysate (SIGMA, Cat. No. 58903C, USA) or 1g/L of yeast extract (SIGMA, Cat. No. Y4375, USA) were added to the culture medium, and the culturing started with a bioreactor containing 1.0 mM of sodium butyrate alone as a control. For the production of hFVII-SOD1 (ATKAVC), the cell line was cultured at 33.0°C for at least 10 days. The supernatant was collected on a daily basis and the cell concentration and the level of cell activity of the cell line were observed.

On the other hand, the supernatant was collected by centrifugation of the culture fluid on a daily basis, and then, effective concentration 50 (EC50) was measured using the COASET Chromogenic assay kit (Chromogenix, Italy). hFVII-SOD1 (ATKAVC), which passed through the final purification process, was diluted from 60 ng/mL to 0.03 ng/mL by a 2-fold serial dilution with a working buffer contained in the kit, and was aliquoted into each well of 96-well plates in an amount of 50 µL, respectively. Then, a combined reagent containing FacX, CaCl₂, and thromboplastin was aliquoted into each well in an amount of 50 µL and reacted at 37°C for 7 minutes. Subsequently, the substrate was aliquoted into each well in an amount of 50 µL. Finally, absorbance was measured at 405 nm and EC50 of each FacVII-SOD1 (ATKAVC) was compared and analyzed (Table 4).

**[Table 4]**

| Result of hFVII-SOD1 (ATKAVC) potency analysis based on the use of additives | |
|---|---|
| Additives | Relative Activity Ratio (% compared to control) |
| Control | 100.0 |
| Soy hydrolysate (1g/L) | 76.6 |
| Yeast extract (1g/L) | 100.0 |

As indicated in Table 4, it was confirmed that the hFVII-SOD1 (ATKAVC) activity was decreased when 1g/L of soy hydrolysate was added and the hFVII-SOD1 (ATKAVC) activity was maintained 100% when 1g/L of yeast extract was added and the production level was stimulated. That is, it was confirmed that the addition of yeast extract not only increased the production level of hFVII-SOD1 (ATKAVC), which was the protein of interest, but also maintained its activity at 100%.

Accordingly, an experiment on measuring the cell line growth and hFVII-SOD1 (ATKAVC) production level according to the amount of yeast extract addition, which was confirmed to be an excellent additive for a culture medium for producing hFVII-SOD1 (ATKAVC), was conducted.

### 6-4: Measurement of the growth of a cell line according to the yeast extract concentration in a production medium and the production level of hFVII-SOD1 (ATKAVC)

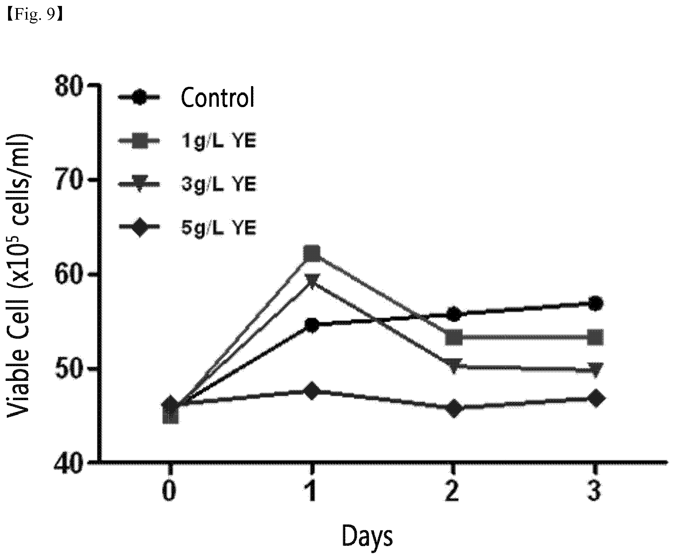

In order to examine the effect of the yeast extract concentration in a production medium on the hFVII-SOD1(ATKAVC) production level, about 10 × 10⁵ cells/mL of recombinant CHO cell lines, which were under cultivation, were inoculated into a 250 mL flask at a culture volume of 50 mL. When the desired cell concentration of 15 x 10⁶ cells/mL was reached, 1.0 mM of sodium butyrate (SIGMA, Cat. No. B5887, USA), and 1g/L, 3g/L, and 5g/L of yeast extract (SIGMA, Cat. No. Y4375, USA) were added to the culture medium, and the culturing started with a bioreactor containing 1.0 mM of sodium butyrate alone as a control. For the production of hFVII-SOD1 (ATKAVC), the cell line was cultured at 33.0°C for at least 3 days. The supernatant was collected on a daily basis and the cell concentration of the cell line was observed (Fig. 9).

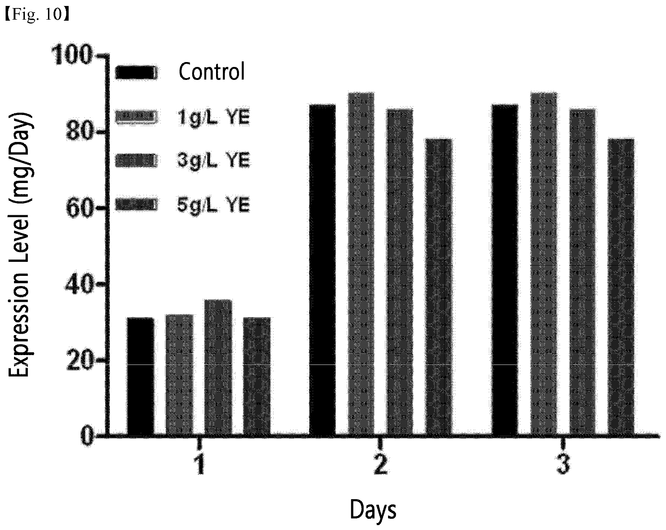

On the other hand, the supernatant was collected by centrifugation on a daily basis, and then, hFVII-SOD1 (ATKAVC) expressed in each supernatant was measured by ELISA assay, as disclosed in Example 3-2. The culture profile and measured values of the bioreactor were calculated and expressed (Fig. 10).

### Example 7 : Method for purifying factor VII derivatives in a culture medium

An ultrafiltration membrane (SARTOCON Slice Cassette, PESU, Sartorius, MWCO 30 K) was washed for at least 1 hour with 1 L of 1 N NaOH, and washed with 5 L of sterilized water. Then, the ultrafiltration membrane was equilibrated with a primary purification column equilibration buffer, and the FVII derivative-producing culture broth was injected into the membrane and concentrated 10-fold. The concentrated culture broth was diluted two-fold with the same volume of the primary purification column equilibration buffer and then concentrated again to its original concentration volume. This dilution-concentration procedure was repeated at least seven times to diafiltrate the culture broth with the primary purification column equilibration buffer. Herein, there was almost no loss of the FVII derivates and the final concentration of concentrated FVII derivatives was maintained at a volume of 0.3 mg/mL. The ultrafiltration and diafiltration were performed at 4°C. The diafilterd FVII derivative culture fluid was finally filtered through a 0.22 µm-filter (NALGENE, PES) and loaded to an anion exchange chromatography. In the present invention, the column filled with Q Sepharose resin (High Performance, GE Healthcare) was used for the anion exchange chromatography. The loaded buffer eluted the protein using equilibration buffer A (20 mM Tris pH8.0 + 2 mM benzamidine), wash buffer B (20 mM Tris pH8.0 + 2 mM benzamidine + 0.2 M NaCl), wash buffer C (20 mM Tris pH8.0 + 2 mM benzamidine + 0.1 M NaCl), and elution buffer D (20 mM Tris pH8.0 + 2 mM benzamidine + 25 mM NaCl + 35 mM CaCl₂) by a linear concentration gradient ranging from wash buffer C to elution buffer D over 2.5 columns. Immediately after the elution, the protein eluate containing FVII derivatives eluted by the anion exchange chromatography was buffer-exchanged by size exclusion chromatography with 20 mM Tris pH 8.0 + 2 mM benzamidine buffer. The filtration, the anion exchange chromatography, and the size exclusion chromatography were performed at 4°C. The sample, which was buffer-exchanged by the size exclusion chromatography, was loaded to the anion exchange chromatography column. In the present invention, the column filled with Q Sepharose resin (High Performance, GE Healthcare) was used. The loaded buffer eluted the protein using equilibration buffer A (20 mM Tris pH 8.0 + 2 mM benzamidine) and elution buffer B (20 mM Tris pH 8.0 + 2 mM benzamidine + 1 M NaCl) by a linear concentration gradient having elution buffer B concentration ranging from 20% to 35% over 15 column volumes. The eluted FVII derivative protein was formulated with 20 mM potassium phosphate buffer (pH 5.5) through the size exclusion chromatography, and all the procedures were performed at 4°C. As described above, FVII derivates may be stored after purification into an inactive form. If necessary, the FVII derivatives may be converted into the active FVIIa derivatives, as described in Example 8.

### Example 8 : Activation of purified FVII derivatives

To activate FVII derivative proteins, the purified FVII derivative sample was loaded to an anion exchange chromatography column previously equilibrated with equilibration buffer A (20 mM Tris pH 8.0 + 2 mM benzamidine). For the anion exchange chromatography, the column filled with Source 15Q resin (GE Healthcare) was used. The loaded buffer was subjected to on-column activation process for 40 minutes using equilibration buffer A and elution buffer B (20 mM Tris pH8.0 + 2 mM benzamidine + 25 mM NaCl + 35 mM CaCl₂) at a concentration of 5%, followed by isocratic elution method using elution buffer B to elute the proteins. The activation of the purified FVII derivatives was performed at room temperature.

From the foregoing, a skilled person in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents and other embodiments that may be included within the spirit and scope of the present invention as defined by the appended claims.
## Claims

1. A method for mass production of human coagulation factor VII derivatives, comprising:
a) constructing an expression vector, comprising i) a nucleotide sequence of a dihydrofolate reductase promoter devoid of at least one CCGCCC repeat sequence from the GC-rich region thereof and a nucleotide sequence encoding a dihydrofolate reductase (DHFR) operably linked thereto, and ii) a nucleotide sequence of a cytomegalovirus (CMV) early gene promoter and a nucleotide sequence encoding a human coagulation factor VII derivative operably linked thereto;
b) transfecting an animal cell line with the expression vector of step a);
c) culturing the transfected animal cell line of step b) in the presence of a dihydrofolate reductase inhibitor to select a cell line capable of expressing the human coagulation factor VII derivative with high efficiency; and
d) culturing the animal cell line selected from step c) by adding at least one selected from the group consisting of sodium butyrate, vitamin K, and a culture medium supplement, wherein:
when sodium butyrate is added: (i) the concentration of sodium butyrate is from 1.1 mM to 3.5 mM and the culture temperature of step d) is from 32.5°C to 35.0°C; or (ii) the concentration of sodium butyrate is from 1.2 mM to 2.0 mM and the culture temperature of step d) is from 31°C to 32.5°C;
the culture medium supplement is soy hydrolysate or yeast extract;
the human coagulation factor VII derivative has a partial sequence of SOD1 (superoxide dismutase 1) at the C-terminus of human coagulation factor VII; and
vitamin K of step d) is vitamin K1.

2. The method of claim 1, wherein:
(i) the GC-rich region comprises one or less CCGCCC repeat sequence;
(ii) the nucleotide sequence encoding the human coagulation factor VII derivative has a nucleotide sequence of SEQ ID NO: 8; or
(iii) the expression vector is pXOGC-FVII described in FIG. 1.

3. The method of claim 1, wherein the animal cell line of step b) is selected from the group consisting of a Chinese hamster ovary (CHO) cell line, a monkey kidney cell line (COS7), an NSO cell line, an SP2/0 cell line, a W138, a baby hamster kidney (BHK) cell line, MDCK, a myeloma cell line, an HuT 78 cell line, and a 293 cell line.

4. The method of claim 3, wherein the animal cell line is deficient in dihydrofolate reductase gene.

5. The method of claim 1, wherein the selected cell line of step c) is HMF709 (Accession No. KCTC 12022BP).

6. The method of claim 1, wherein the concentration of sodium butyrate is 1.5 mM when the culture temperature of step d) is 33.0°C.

7. The method of any one of claims 1 to 5, wherein
vitamin K of step d) is in a concentration from 0.1 mg/L to 3 mg/L.

8. The method of claim 1, wherein:
(i) the culture medium supplement of step d) is yeast extract; or
(ii) soy hydrolysate or yeast extract in a concentration from 0.1 g/L to 3 g/L is added to the culture medium.

9. The method of claim 1, which further comprises (a) purifying human coagulation factor VII derivative; or (b)activating the human coagulation factor VII derivative.

10. The method of claim 1, wherein, in step d), the animal cell line selected from step c) is cultured in the presence of soy hydrolysate or yeast extract.

11. The method of claim 1, wherein ,in step d), the animal cell line selected from step c) is cultured in the presence of yeast extract.

12. The method of claim 1, wherein the partial sequence of SOD1 is ATKAVC (SEQ ID NO: 5).

13. A method for mass production of human coagulant factor VII derivatives comprising culturing an HMF709 cell line (Accession No. KCTC 12022 BP), capable of producing human coagulation factor VII derivatives by adding at least one selected from the group consisting of sodium butyrate, vitamin K, and a culture medium supplement;
wherein the culture medium supplement is soy hydrolysate or yeast extract; and vitamin K is vitamin K1;
wherein when sodium butyrate is added: (i) the concentration of sodium butyrate is from 1.1 mM to 3.5 mM and the culture temperature is from 32.5°C to 35.0°C; or (ii) the concentration of sodium butyrate is from 1.2 mM to 2.0 mM and the culture temperature is from 31°C to 32.5°C.

## Patentansprüche

1. Verfahren zur Massenherstellung von Derivaten von menschlichem Gerinnungsfaktor VII, Folgendes umfassend:
a) Konstruktion eines Expressionsvektors, umfassend i) eine Nucleotidsequenz eines Dihydrofolatreduktase-Promotors, dem zumindest eine CCGCCC-Wiederholungssequenz aus der CG-reichen Region davon fehlt, und eine operabel daran gebundene Nucleotidsequenz, die für eine Dihydrofolatreduktase (DHFR) kodiert, und ii) eine Nucleotidsequenz eines frühen Cytomegalievirus- (CMV-) Genpromotors und eine operabel daran gebundene Nucleotidsequenz, die für ein Derivat von menschlichem Gerinnungsfaktor VII kodiert;
b) Transfizieren einer Tierzelllinie mit dem Expressionsvektor von Schritt a);
c) Kultivieren der transfizierten Tierzelllinie von Schritt b) in Gegenwart eines Dihydrofolatreduktase-Hemmers zum Auswählen einer Zelllinie, die in der Lage ist, hocheffizient ein Derivat von menschlichem Gerinnungsfaktor VII zu exprimieren; und
d) Kultivieren der in Schritt c) ausgewählten Zelllinie durch Zusetzen von zumindest einem, das aus der Gruppe, die aus Natriumbutyrat, Vitamin K und einem Kulturmediumergänzungsmittel besteht, ausgewählt ist, wobei
wenn Natriumbutyrat zugesetzt wird, (i) die Natriumbutyratkonzentration 1,1 mM bis 3,5 mM beträgt und die Kulturtemperatur von Schritt d) 32,5°C bis 35,0 °C beträgt; oder (ii) die Natriumbutyratkonzentration 1,2 mM bis 2,0 mM beträgt und die Kulturtemperatur von Schritt d) 31 °C bis 32,5 °C beträgt;
das Kulturmediumeränzungsmittel Sojahydrolysat oder Hefeextrakt ist;
das Derivat von menschlichem Gerinnungsfaktor VII eine Teilsequenz von SOD1 (Superoxiddismutase 1) am C-Terminus von menschlichem Gerinnungsfaktor VII aufweist; und
das Vitamin K von Schritt d) Vitamin K1 ist.

2. Verfahren nach Anspruch 1, wobei:
(i) die GC-reiche Region eine oder weniger CCGCCCC-Wiederholungssequenzen umfasst;
(ii) die für das Derivat von menschlichem Gerinnungsfaktor VII kodierende Nucleotidsequenz eine Nucleotidsequenz von SEQ ID NO: 8 aufweist; oder
(iii) der Expressionvektor pXOGC-FVII ist, wie in FIG. 1 beschrieben.

3. Verfahren nach Anspruch 1, wobei die Tierzelllinie von Schritt b) aus der Gruppe ausgewählt ist, die aus einer Chinahamster-Eierstockzell- (CHO-) Linie, einer Affennieren-Zelllinie (COS7), einer NSO-Zelllinie, einer SP2/0-Zelllinie, W138, einer Babyhamsternieren- (BHK-) Zellinie, MDCK, einer Myelomzelllinie, einer HuT78-Zelllinie und einer 293-Zelllinie besteht.

4. Verfahren nach Anspruch 3, wobei die Tierzelllinie an Dihydrofolatreduktase-Gen verarmt ist.

5. Verfahren nach Anspruch 1, worin die ausgewählte Zelllinie von Schritt c) HMF709 (Zugrifssnr. KCTC 12022BP) ist.

6. Verfahren nach Anspruch 1, wobei die Konzentration von Natriumbutyrat 1,5 mM beträgt, wenn die Kulturtemperatur von Schritt d) 33,0 °C beträgt.

7. Verfahren nach einem der Ansprüche 1 bis 5, wobei
das Vitamin K von Schritt d) in einer Konzentration von 0,1 mg/l bis 3 mg/l vorliegt.

8. Verfahren nach Anspruch 1, wobei
(i) das Kulturmediumergänzungsmittel von Schritt d) Hefeextrakt ist; oder
(ii) Sojahydrolysat oder Hefeextrakt in einer Konzentration von 0,1 g/l bis 3 g/l zum Kulturmedium zugesetzt wird.

9. Verfahren nach Anspruch 1, ferner umfassend (a) Reinigen von Derivat von menschlichem Gerinnungsfaktor VII; oder (b) Aktivieren von Derivat von menschlichem Gerinnungsfaktor VII.

10. Verfahren nach Anspruch 1, wobei in Schritt d) die ausgewählte Tierzelllinie von Schritt c) in Gegenwart von Sojahydrolysat oder Hefeextrakt kultiviert wird.

11. Verfahren nach Anspruch 1, wobei in Schritt d) die ausgewählte Tierzelllinie von Schritt c) in Gegenwart von Hefeextrakt kultiviert wird.

12. Verfahren nach Anspruch 1, wobei die Teilsequenz von SOD1 ATKACV (SEQ ID NO: 5) ist.

13. Verfahren zur Massenherstellung von Derivaten von menschlichem Gerinnungsfaktor VII, umfassend das Kultivieren einer HMF709-Zelllinie (Zugriffsnr. KCTC 12022 BP), die in der Lage ist, Derivate von menschlichem Gerinnungsfaktor VII zu erzeugen, durch Zusetzen von zumindest einem, das aus der Gruppe, die aus Natriumbutyrat, Vitamin K und einem Kulturmediumergänzungsmittel besteht, ausgewählt ist;
wobei das Kulturmediumergänzungsmittel Sojahydrolysat oder Hefeextrakt ist; und das Vitamin K Vitamin K1 ist;
wobei, wenn Natriumbutyrat zugesetzt wird, (i) die Natriumbutyratkonzentration 1,1 mM bis 3,5 mM beträgt und die Kulturtemperatur 32,5 °C bis 35,0 °C beträgt; oder (ii) die Natriumbutyratkonzentration 1,2 mM bis 2,0 mM beträgt und die Kulturtemperatur 31 °C bis 32,5 °C beträgt.

## Revendications

1. Procédé de production en masse de dérivés de facteur VII de coagulation humain, comprenant les étapes consistant à :
a) construire un vecteur d'expression, comprenant i) une séquence nucléotidique d'un promoteur de dihydrofolate réductase dépourvue d'au moins une séquence de répétition CCGCCC de sa région riche en GC et une séquence nucléotidique codant pour une dihydrofolate réductase (DHFR) liée de manière fonctionnelle à celle-ci, et ii) une séquence nucléotidique d'un promoteur de gène précoce du cytomégalovirus (CMV) et une séquence nucléotidique codant pour un dérivé du facteur VII de coagulation humain lié de manière fonctionnelle à celle-ci ;
b) transfecter une lignée cellulaire animale avec le vecteur d'expression de l'étape a) ;
c) cultiver la lignée cellulaire animale transfectée de l'étape b) en présence d'un inhibiteur de dihydrofolate réductase afin de sélectionner une lignée cellulaire capable d'exprimer le dérivé de facteur VII de coagulation humain avec une grande efficacité ; et
d) cultiver la lignée cellulaire animale sélectionnée à l'étape c) en ajoutant au moins un sélectionné dans le groupe comprenant le butyrate de sodium, la vitamine K et un supplément de milieu de culture, dans lequel :
lorsque du butyrate de sodium est ajouté : (i) la concentration en butyrate de sodium est comprise entre 1,1 mM et 3,5 mM et la température de culture de l'étape d) est comprise entre 32,5°C et 35,0°C ; ou (ii) la concentration en butyrate de sodium est comprise entre 1,2 mM et 2,0 mM et la température de culture de l'étape d) est comprise entre 31°C et 32,5°C ;
le complément de milieu de culture est un hydrolysat de soja ou un extrait de levure ;
le dérivé de facteur VII de coagulation humain a une séquence partielle de SOD1 (superoxyde dismutase 1) à l'extrémité C-terminale du facteur VII de coagulation humain ; et
la vitamine K de l'étape d) est la vitamine K1.

2. Procédé selon la revendication 1, dans lequel :
(i) la région riche en GC comprend une séquence de répétition CCGCCC ou moins ;
(ii) la séquence nucléotidique codant pour le dérivé de facteur VII de coagulation humain a une séquence nucléotidique de SEQ ID NO : 8 ; ou
(iii) le vecteur d'expression est pXOGC-FVII décrit sur la figure 1.

3. Procédé selon la revendication 1, dans lequel la lignée cellulaire animale de l'étape b) est choisie dans le groupe constitué d'une lignée cellulaire d'ovaire de hamster chinois (CHO), d'une lignée cellulaire de rein de singe (COS7), d'une lignée cellulaire NSO, d'une lignée cellulaire SP2/0, d'une lignée W138, d'une lignée cellulaire de rein de bébé hamster (BHK), de MDCK, d'une lignée cellulaire de myélome, d'une lignée cellulaire HuT 78 et d'une lignée cellulaire 293.

4. Procédé selon la revendication 3, dans lequel la lignée cellulaire animale est déficiente en gène de dihydrofolate réductase.

5. Procédé selon la revendication 1, dans lequel la lignée cellulaire sélectionnée de l'étape c) est HMF709 (numéro d'enregistrement KCTC 12022BP).

6. Procédé selon la revendication 1, dans lequel la concentration en butyrate de sodium est de 1,5 mM lorsque la température de culture de l'étape d) est de 33,0°C.

7. Procédé selon l'une quelconque des revendications 1 à 5, dans lequel
la vitamine K de l'étape d) est à une concentration de 0,1 mg/L à 3 mg/L.

8. Procédé selon la revendication 1, dans lequel :
(i) le supplément de milieu de culture de l'étape d) est un extrait de levure ; ou
(ii) de l'hydrolysat de soja ou de l'extrait de levure à une concentration de 0,1 g/L à 3 g/L est ajouté au milieu de culture.

9. Procédé selon la revendication 1, qui comprend en outre : (a) la purification du dérivé de facteur VII de coagulation humain ; ou (b) l'activation du dérivé de facteur VII de coagulation humain.

10. Procédé selon la revendication 1, dans lequel, à l'étape d), la lignée cellulaire animale sélectionnée à l'étape c) est cultivée en présence d'hydrolysat de soja ou d'extrait de levure.

11. Procédé selon la revendication 1, dans lequel, à l'étape d), la lignée cellulaire animale sélectionnée à l'étape c) est cultivée en présence d'extrait de levure.

12. Procédé selon la revendication 1, dans lequel la séquence partielle de SOD1 est ATKAVC (SEQ ID NO : 5).

13. Procédé de production en masse de dérivés de facteur VII coagulant humain, comprenant la culture d'une lignée cellulaire HMF709 (numéro d'enregistrement KCTC 12022 BP), capable de produire des dérivés de facteur VII de coagulation humain par addition d'au moins un sélectionné dans le groupe comprenant le butyrate de sodium, la vitamine K et un supplément de milieu de culture ;
dans lequel le supplément de milieu de culture est un hydrolysat de soja ou un extrait de levure ; et la vitamine K est la vitamine K1 ;
dans lequel, lorsque du butyrate de sodium est ajouté : (i) la concentration en butyrate de sodium est comprise entre 1,1 mM et 3,5 mM, et la température de culture est comprise entre 32,5°C et 35,0°C ; ou (ii) la concentration en butyrate de sodium est comprise entre 1,2 et 2,0 mM, et la température de culture est comprise entre 31°C et 32,5°C.
